# EUROPEAN PATENT APPLICATION

(11) **EP 1 167 516 A2**
(43) Date of publication of application: **02.01.2002**
(21) Application number: 01106704.8
(22) Date of filing: 16.03.2001
(51) Int. Cl.: C12N 1/26, C12R 1/01, C02F 3/34

(54) **Aromatic compound-degrading bacteria and use thereof**

(30) Priority: 26.06.2000 JP 2000190560
(71) Applicant: Sumitomo Forestry Co., Ltd., Osaka-shi, Osaka 541 (JP)
(72) Inventor: Oshiman, Koichi, c/oSumitomo Forestry Co.,Ltd., Osaka-shi, Osaka (JP); Soda, Ryo, c/oSumitomo Forestry Co.,Ltd., Osaka-shi, Osaka (JP); Oishi, Akiko, c/oSumitomo Forestry Co.,Ltd., Osaka-shi, Osaka (JP)
(74) Representative: Henkel, Feiler, Hänzel

(57) **Abstract**

A novel bacterium Sphingomonas sp. A0-1 (FERM BP-7435) belonging to the genus Sphingomonas was isolated from microorganisms grown by culturing sources for isolation such as soils or activated sludges in a medium using bisphenol A as the sole carbon source.
The bacterium can efficiently degrade bisphenol A and an aromatic compound containing a stilbene skeleton and can thus be used for environmental cleanup.

## Description

### BACKGROUND OF THE INVENTION

### FIELD OF THE INVENTION

The present invention relates to novel bacteria and a method for environmental purification using the same. More particularly, the invention relates to bacteria capable of degrading at least one aromatic compound selected from bisphenol A and aromatic compounds having a stilbene skeleton as well as a method for degradation of aromatic compounds in the soils, waters or other wastes suspected of containing bisphenol A or aromatic compounds having a stilbene skeleton, comprising allowing the bacteria to act on the soils, waters or other wastes, which can be used for environmental purification.

### DESCRIPTION OF RELATED ARTS

In recent years, it is concerned that with the increased industrial applications of resin products like plastics, water pollution is caused by an outflow of substances having an endocrine disruputing influence such as bisphenol A, etc., which are suspected of a serious influence like teratogenesis. Such tetratogenesis would adversely affect the next generation. For example, bisphenol A dissolves out of plastic products or cans for drinks to affect cell division of the early embryo of fertilized ova in mice even in a concentration as low as 0.228 ppb (the Mainichi newspaper dated June 17, 1999). Furthermore, examination of offspring by consecutively administering bisphenol A-containing feed to pregnant mice for a week indicates that in the 2 ppb group atrophy of the epididymides was observed and sperm producing ability decreased in the 20 ppb group (the Mainichi newspaper dated May 27, 1998). On the other hand, since bisphenol A was reported to dissolve out of plastic containers by hot water, polycarbonate-made plastic tableware used for school lunch has now been cleared away. River pollution survey by the Ministry of Construction reveals that endocrine-disrupting chemicals (EDCS) were detected at 147 out of 256 spots, indicating that rivers in Japan are polluted over a wide area. Reportedly, the maximum level of bisphenol A in the rivers reached 1.4 ppb (the Mainichi newspaper dated October 26, 1998). The Ministry of Construction announced that construction of waste disposal plants will be considered in the future to reduce endocrine-disrupting chemicals (EDCs) (the Mainichi newspaper dated June 23, 1999). It was also reported that a high level of bisphenol A at the maximum of 17.2 ppm was contained in the water seeped from waste disposal facilities and these waste substances including bisphenol A were dissolved away from a part of the waste plastic products in those facilities (the Kobe newspaper dated May 31, 1998). Endocrine-disrupting chemicals (EDCs) contain in part agricultural chemicals, sources of which are reportedly farmland and golf courses.

For removal of these pollutants widely spread into the aquatic system or soils, biodegradation with microorganisms is considered to be most practical (Chikyu-Ga-Yomigaeru (A Revival of the Earth), published by CMC). However, any technology for removing such pollutant has not yet been established on a practical field-working level.

Only two strains are known so far as bacteria for degrading bisphenol A, one of which is an unidentified bacterium designated as strain MVI (Applied and Environmental Microbiology, 58: 1823-1831, 1992) and the other is Pseudomonus paucimobilis FJ-4, later named Sphingomonas paucimobilis (Japanese Journal of Water Treatment Biology, 31: 203-212, 1995). The strain MV1 was disadvantageous because biodegradation of bisphenol A by MV1 under the conditions later described accumulated 4-hydroxyacetophenone to 1.5 mM or more, at which level MV1 could no longer proliferate. Furthermore, sparingly biodegradable 4-hydoxy-3-methylacetophenone, 4-hydroxy-3-methylbenzoic acid and 2,2-bis(4-hydroxy-3-methylphenyl)-1-propanol were by-produced and accumulated in the culture medium (Applied and Environmental Microbiology, 58: 1823-1831, 1992). Turning to the strain FJ4, the strain could not biodegrade bisphenol A at the saturated concentration (ca. 1.5 mM) but could merely degrade 1 mM of bisphenol A at the maximum as the initial concentration.

In view of the disadvantages noted in the prior art, it is desired to develop a microorganism suitable for biological purification that not only has an activity sufficient to degrade bisphenol A, etc. but can fully degrade and remove aromatic compounds such as bisphenol A without by-producing residual intermediate metabolites. Furthermore, the density of applied microorganisms having a biodegradable activity tends to be lowered in the environments due to predation by protozoan or by competition with other microorganisms so that the capability of microorganisms necessary for biodegradation cannot be often exerted. Even though a microorganism capable of degrading a target substance is introduced into the environment, it is likely that the microorganism applied might be killed by other organic solvents, etc. contained in water, without exhibiting its activity for degrading the target substance. It is thus desired to find bacteria having a high value-added property, e.g., resistance to organic solvent, etc.

In view of the requirements above, it has been attempted not only to isolate a highly active microorganism from the nature but also to cultivate a microorganism having a high biodegradability and excellent environmental adaptability, making full use of genetic engineering technology. It is now concerned, however, that when particular microorganisms, especially microorganisms produced by genetic recombinant technology, are released in the environment, they might potentially affect the ecosystem. Therefore, if a target substance can be degraded by sterilized bacteria, the microorganisms will be applicable to wide areas.

### SUMMARY OF THE INVENTION

The present invention provides a novel microorganism capable of degrading bisphenol A or aromatic compounds containing a stilbene skeleton more efficiently than hitherto known microorganisms, as well as a method for degradation of bisphenol A or aromatic compounds containing a stilbene skeleton using the same.

The present inventors made various investigations to solve the foregoing problems and as a result, have succeeded in isolating a novel microorganism having a much higher activity of degrading bisphenol A or aromatic compounds containing a stilbene skeleton than that of known microorganisms. The novel microorganism does not belong to any of the known species. The present invention has thus been accomplished.

Therefore, the present invention relates to novel bacteria capable of degrading at least one of bisphenol A and an aromatic compound containing a stilbene skeleton, which bacteria belong to the genus Sphingomonas but correspond to no other known species. More specifically, the bacteria are characterized by the following properties.

| | |
|---|---|
| Morphology | rod |
| Gram staining | - |
| Spore formation | - |
| Motility | + |
| Behavior to oxygen | aerobic |
| Oxidase test | + |
| Catalase test | + |
| OF | - |
| Color of colonies | yellowish |

A representative strain is Sphingomonas sp. A0-1 (FERM BP-7438). As the aromatic compounds containing a stilbene skeleton, there are, for example, trans-stilbene, stilbene dyes (Chrysophenine G, etc.) and the like. The present invention further relates to a method for degradation of such aromatic compounds which comprises allowing the bacteria to act on at least one aromatic compound selected from bisphenol A and aromatic compounds containing a stilbene skeleton.

The present invention further relates to a method for degradation of an aromatic compounds in the soils, waters or other wastes suspected of containing at least one of bisphenol A and an aromatic compound containing a stilbene skeleton which comprises adding to the soils, waters or other wastes a cultured bacterial mass or a carrier to which a bacterial mass has been adhered. The culture of the bacteria may be living or sterilized. Examples of the sterilization include UV irradiation and sterilization by filtration.

### BRIEF DESCRIPTION OF THE DRAWINGS

Fig. 1 shows a taxonomic tree produced by the NJ (proximal conjugation) method by comparing the sequence in the region covering almost 95% of 16S rRNA gene in its full-length of about 1.5 kb from Sphingomonas sp. A0-1 with the sequences of known microorganisms.
Fig. 2 shows the effect of Sphingomonas sp. A0-1, Sphingomonas chlorophenolica ATCC 33790 and Sphingomonas herbicidovorans DSM11019 on bisphenol A level in BSM medium.
Fig. 3 shows the effects of Sphingomonas sp. A0-1, Sphingomonas chlorophenolica ATCC 33790 and Sphingomonas herbicidovorans DSM11019 on bisphenol A level in MYPG medium.
Fig. 4 shows the effect of glucose level in BSM medium on the reduction in bisphenol A level by Sphingomonas sp. A0-1 in the medium.
Fig. 5 shows the effects of sucrose, maltose or dextrin level in BSM medium on the reduction in bisphenol A level by Sphingomonas sp. A0-1 in the medium.
Fig. 6 shows the effect of incubation temperatures on the growth of Sphingomonas sp. A0-1 in MYPG medium.
Fig. 7 shows the effect of medium pH values on the growth of Sphingomonas sp. A0-1 in MYPG medium.
Fig. 8 shows the effects of the added bacterial cell suspension and sterilized cell suspension of Sphingomonas sp. A0-1 on the concentration of bisphenol A in BSM medium and in the bacterial cell extract.

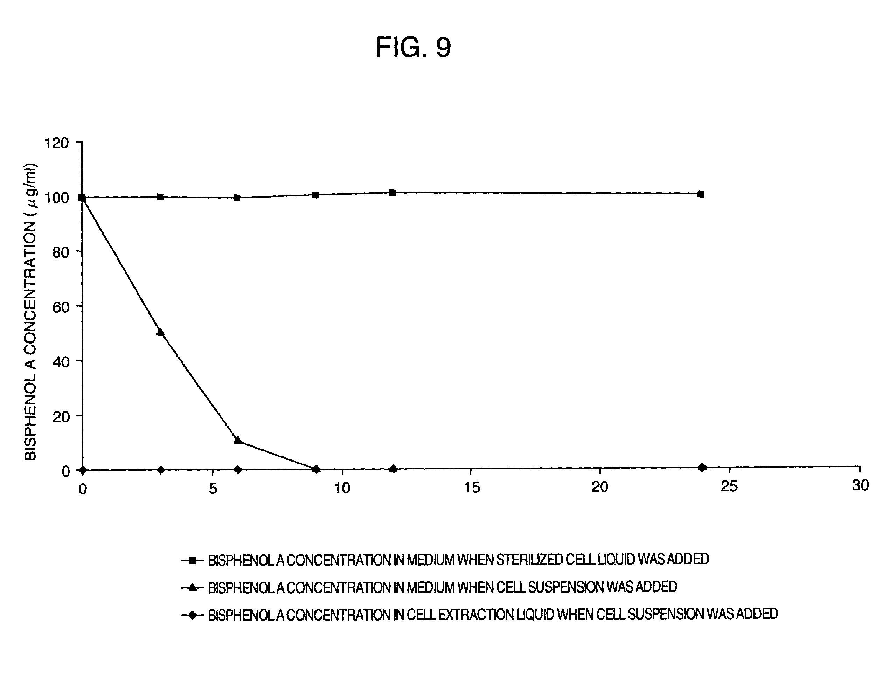

Fig. 9 shows the effects of the added bacterial cell suspension and sterilized cell solution of Sphingomonas sp. A0-1 on the concentration of bisphenol A in MYPG medium and in the bacterial cell extract.
Fig. 10 shows the effect of bisphenol A added to a medium during preincubation on the reduction of bisphenol A level by Sphingomonas sp. A0-1 in BSM medium.
Fig. 11 shows the effect of warming temperatures on the reduction of bisphenol A level by culture filtrate of Sphingomonas sp. A0-1.

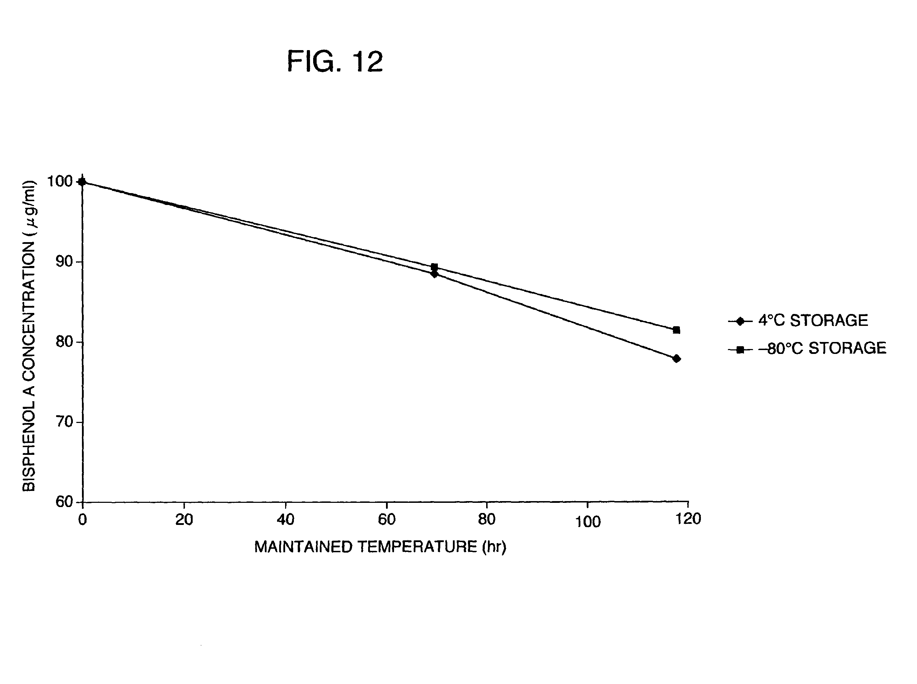

Fig. 12 shows the effects of culture filtrates of Sphingomonas sp. A0-1 on the reduction of bisphenol A level, under cold storage and freeze storage.
Fig. 13 shows a scanning electron microscopic image of Sphingomonas sp. A0-1.

### DETAILED DESCRIPTION OF THE INVENTION

Sphingomonas sp. A0-1 which is a representative strain of the present invention can be isolated using the following enrichment culture method.

For example, the sources for isolation such as soils or activated sludges are cultured in a medium supplemented with 20 µg/mL of bisphenol A as the sole carbon source. The culture medium grown is then cultured in a medium supplemented with 60 µg/mL of bisphenol A and further in a medium supplemented with 100µg/mL of bisphenol A. Subsequently, a microorganism having a degradation activity is selected in an agar medium supplemented with 100µg/mL of bisphenol A as the sole carbon source. The thus isolated Sphingomonas sp. A0-1 has the following taxonomical properties.

| | |
|---|---|
| Morphology | rod |
| Gram staining | - |
| Spore formation | - |
| Motility | + |
| Behavior to oxygen | aerobic |
| Oxidase test | + |
| Catalase test | + |
| OF | - |
| Color of colonies | yellowish |

The strain A0-1 further has the following properties.

| | |
|---|---|
| Growth under anaerobic conditions | - |
| Catalase test | + |
| Oxidase test | + |
| Reduction of nitrate | + |
| V-P test | - |
| pH in V-P broth | 6.91 |
| Hydrolysis of casein | - |
| Liquefaction of gelatin | + |
| Hydrolysis of esculin | - |
| Hydrolysis of starch | - |
| Decomposition of DNA | - |
| Degradation of urea | - |
| Degradation of tyrosine | - |
| Degradation of arginine | - |
| Hydrolysis of Tween 20 | - |
| Hydrolysis of Tween 80 | - |
| Utilization of citrate | + |
| Production of Indole | - |
| Production of hydrogen sulfide | - |
| Growth in the presence of NaCl | - |
| 5% | - |
| 7% | - |
| pH for growth | 5.5 - 8.5 |
| Growth temperature (°C) | 10 - 40 |
| Growth in the presence of 0.01% lysozyml | - |

Regarding the production of acid from the following sugars, the strain A0-1 has the properties shown below.

| | |
|---|---|
| Glucose | + |
| Arabinose | + |
| Fructose | - |
| Galactose | + |
| Maltose | + |
| Lactose | - |
| Sucrose | + |
| Xylose | + |
| Trehalose | + |
| Glycerol | - |
| Mannitol | - |
| Cellobiose | + |
| Ribose | - |
| Salicin | - |
| Sorbitol | - |
| Sorbose | - |
| Mannose | - |
| Melibiose | - |
| Rhamnose | + |
| Raffinose | - |
| Inositol | - |
| Adonitol | - |
| Formation of gas from Glucose | - |

The strain was named Sphingomonas sp. A0-1 and deposited on March 23, 2000 at the National Institute of Bioscience and Human Technology Agency of Industrial Science and Technology and FERM P-17794 as an acession number. Then, the deposition was transferred to an international deposition under the Budapest Treaty on January 25, 2001 and given FERM BP-7435 as an acession number.

Bisphenol A or the aromatic compounds containing a stilbene skeleton can be degraded by acting the bacteria of the present invention on these compounds.

According to the present invention, the aromatic compounds can be decomposed by adding the cultured bacterial mass of the invention or the bacterial mass-bearing carrier to the soils, waters or other wastes suspected of containing such compounds. As the bacterial mass, there may be employed the culture medium per se obtained by culturing the bacteria of the invention, the cultured bacterial mass isolated from the culture medium, or cultured medium in which bacteria were killed, or killed isolated bacterial mass, etc. To prepare the cultured bacterial mass from the culture medium, the bacterial mass may also be separated by means of conventional centrifugation followed by lyophilization or drying. In order to dry the bacterial mass, lyophilization or spray drying may be used. To minimize influence on environments, sterilized bacterial mass may also be used. Sterilization includes sterilization by filtration, UV irradiation, a treatment with ethylene oxide, radiation sterilization, etc. In the present invention, the compounds contained in the soils, waters or other wastes can be degraded by inoculating a carrier to which the bacterial mass is adhered onto the media to be treated and proliferating the bacteria therein. A porous carrier such as charcoal is preferred as the bacterial mass-bearing carrier.

Where the cultured bacterial mass of the invention is added to the media to be treated, such as the soils, waters or other wastes, the cultured bacterial mass is preferably used in an amount corresponding to 10⁴ to 10¹⁰ living or sterilized bacterial cells/g based on the media to be treated. To allow the bacteria of the invention to act on bisphenol A or the aromatic compounds containing a stilbene skeleton contained in solid media such as soils or in waters, the cultured bacterial mass of the invention or the bacterial mass-bearing carrier may be added to or mixed with the media to be treated.

For culturing the bacteria of the invention, an optional medium may be used so long as the bacteria can grow in the medium. The medium may contain, e.g., glucose, sucrose, etc. as carbon sources, yeast extract, peptone, meat extract, etc. as organic nitrogen sources and ammonium salts, nitrates, etc. as inorganic nitrogen sources. The medium may also contain inorganic salts of cations such as potassium, sodium, calcium or magnesium ions and anions such as chlorine, sulfate or phosphate ions. The concentration of the carbon sources may vary depending upon kind but is generally in the range of approximately 0.1 to 1%. Likewise, the concentration of nitrogen sources may vary depending upon kind but is generally in the range of approximately 0.01 to 1%. The concentration of inorganic salts may also vary depending upon kind but is generally in the range of approximately 0.001 to 0.1%. Culture is preferably by liquid culture under aerobic conditions. The aerobic conditions are provided in a conventional manner by aeration, stirring, combination of aeration and stirring, shaking, etc. As the sole carbon source, bisphenol A or the aromatic compound containing a stilbene skeleton may be employed. The amount of bisphenol A or the aromatic compound containing a stilbene skeleton in a medium is preferably in the range of approximately 1 ppm to 1000 ppm.

Hereinafter the invention will be described in more detail, with reference to EXAMPLES below.

### EXAMPLE 1

### Isolation and identification of strain A0-1

### 1) Isolation of strain A0-1

The strain A0-1 of the invention was isolated as follows. Samples of about 100 ml each collected from field soils and riverbed sludges in Ibaraki Prefecture were put in a vinyl bag, respectively, which were stored in a refrigerator to provide for test in a day or two. The sludge was added in an amount of 1 g to a BSM medium (1.0 g/L K₂HPO₄, 1.0 g/L (NH₄)₂SO₄, 0.2 g/L MgSO₄·7H₂O, 0.01 g/L FeCl₃, 0.05 g/L NaCl and 0.05 g/L CaCl₂) supplemented with 20 µg/mL of bisphenol A as the sole carbon source, followed by culturing at 30°C for about 7 days. After growth of the bacteria was visually confirmed by the suspended state of the medium, the culture medium was further cultured in BSM medium supplemented with 60 µg/mL of bisphenol A and then in BSM medium supplemented with 100 µg/mL of bisphenol A. Thereafter, the culture medium was smeared on a BSM plate agar medium supplemented with 100 µg/mL of bisphenol A as the sole carbon source and 1.5% agar, followed by culturing at 30°C for 3 to 7 days. About 100 colonies appeared were isolated.

With each of about 100 colonies above, the degradability of bisphenol A was determined by the following method. One platinum loop of colonies was fished from the agar medium when isolated, which was inoculated on 7 ml of BSM medium supplmented with 100 µg/mL of bisphenol A charged in a test tube with a 18 cm diameter. After culturing at 30°C for 3 to 7 days, bisphenol A in the medium was quantitatively determined by the following method.

Using high performance liquid chromatography (Alliance 2690, manufactured by Waters Inc.), bisphenol A was quantitatively determined under the following conditions; column: Mightysil RP18 GP150-4.6 (5 µm), made by Kanto Kagaku K.K., eluate: 70% acetonitrile aqueous solution, column tank temperature: 40°C. Using a photo diode array as a detector, bisphenol A was quantitatively determined at absorbance of 217 nm.

As a result, a strain showing the fastest degradation rate was selected from the isolated colonies of about 100. The strain was named A0-1.

### 2) Identification of strain A0-1

i) Morphological and physiological properties of the strain A0-1 were observed in accordance with the literature (Biseibutsu-no-Bunrui-to-Dotei (Classification and Identification of Microorganism), revised version, vol. II, published by Gakkai Shuppan Center, 1985). The results are shown below.

| | |
|---|---|
| Morphology | rod |
| Gram staining | - |
| Spore formation | - |
| Motility | + |
| Behavior to oxygen | aerobic |
| Oxidase test | + |
| Catalase test | + |
| OF | - |
| Color of colonies | yellowish |

The following properties were further obtained.

| | |
|---|---|
| Growth under anaerobic conditions | - |
| Catalase test | + |
| Oxidase test | + |
| Reduction of nitrate | + |
| V-P test | - |
| pH in V-P broth | 6.91 |
| Hydrolysis of casein | - |
| Liquefaction of gelatin | + |
| Hydrolysis of esculin | - |
| Hydrolysis of starch | - |
| Decomposition of DNA | - |
| Degradation of urea | - |
| Degradation of tyrosine | - |
| Degradation of arginine | - |
| Hydrolysis of Tween 20 | - |
| Hydrolysis of Tween 80 | - |
| Utilization of citrate | + |
| Formation of Indole | - |
| Formation of hydrogen sulfide | - |
| Growth in the presence of NaCl | |
| 2% | - |
| 5% | - |
| 7% | - |
| pH for growth | 5.5 - 8.5 |
| Growth temperature (°C) | 10 - 40 |
| Growth in the presence of 0.01% lysozyml | - |

Regarding the production of acid from the following sugars, the strain A0-1 has the properties shown below.

| | |
|---|---|
| Glucose | + |
| Arabinose | + |
| Fructose | - |
| Galactose | + |
| Maltose | + |
| Lactose | - |
| Sucrose | + |
| Xylose | + |
| Trehalose | + |
| Glycerol | - |
| Mannitol | - |
| Cellobiose | + |
| Ribose | - |
| Salicin | - |
| Sorbitol | - |
| Sorbose | - |
| Mannose | - |
| Melibiose | - |
| Rhamnose | + |
| Raffinose | - |
| Inositol | - |
| Adonitol | - |
| Formation of gas from Glucose | - |

ii) Based on the above results, identification of the strain was made according to the literature (N. R. Krieg and J. G. Holt, "Bergey's Manual of Systematic Bacteriology", Vol. 1 (1984), Williams & Wilkins; and J. G. Holt, N. R. Krieg, P. H. A. Senath, J. T. Staley and S. T. Williams, "Bergey's Manual of Determinative Bacteriology", Ninth Edition (1994), Williams & Wilkins). The results suggest that the strain A0-1 would be a strain belonging to the genus Flavobacterium (currently named the genus Sphingomonas).

For further identification, the sequencing of the microorganism of the invention was determined in the region covering almost 95% of 16S rRNA gene in its full-length of about 1.5 kb. The sequence determined is shown as SEQ ID NO. 1 in Sequence Listing.

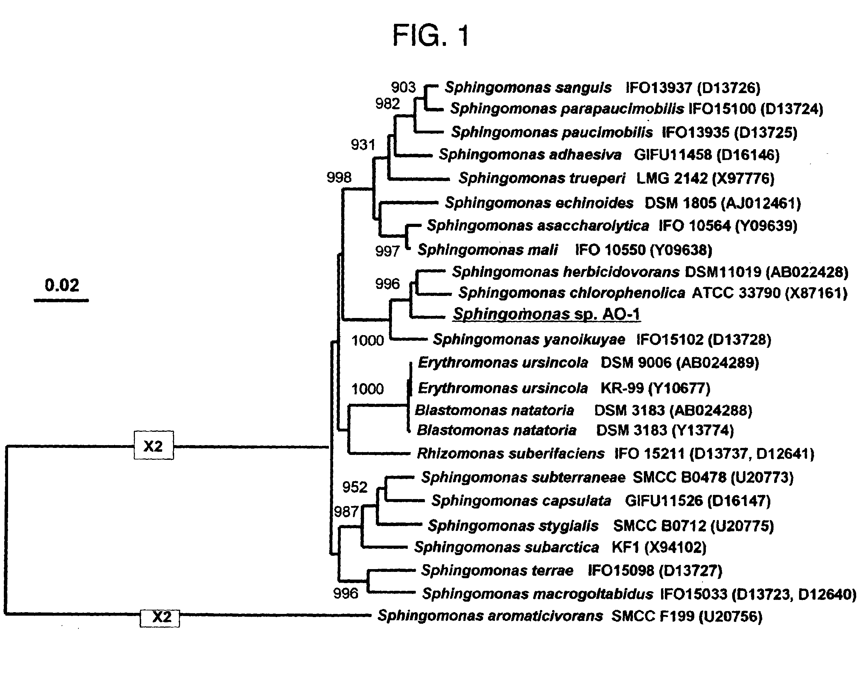

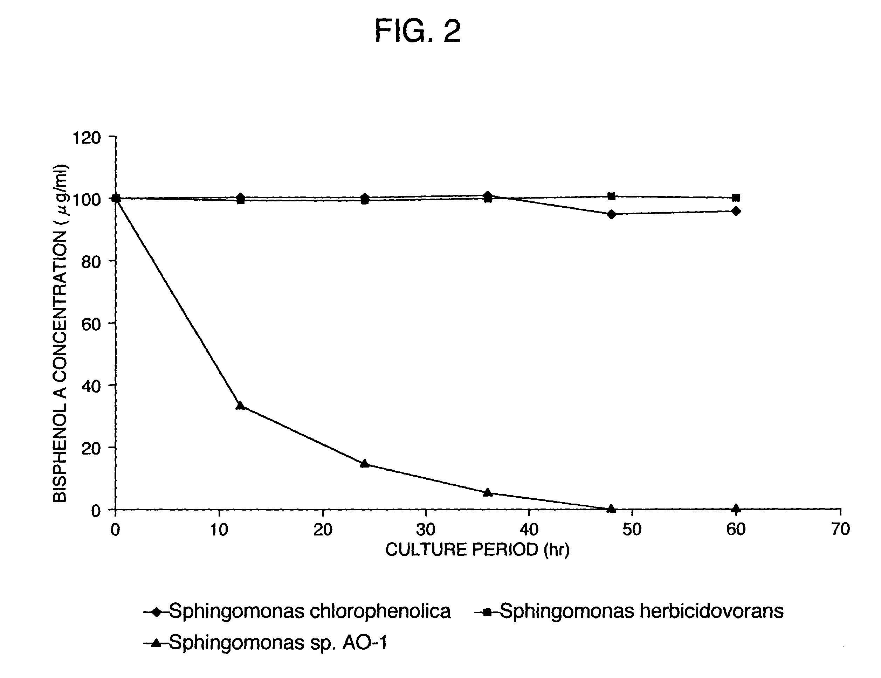

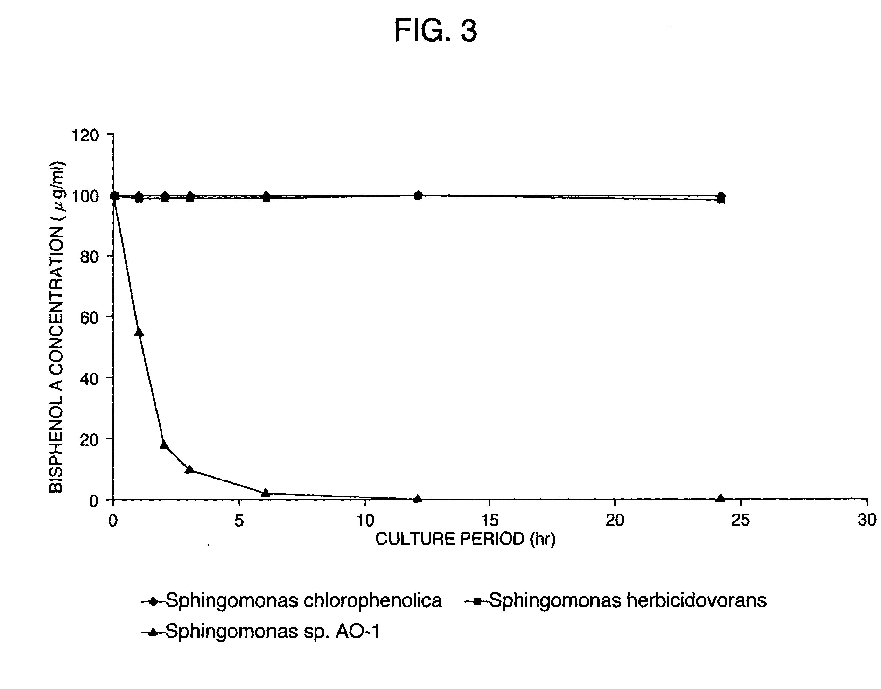

The sequence thus determined was compared to those of known microorganisms to prepare a taxonomic tree according to the NJ method. As shown in Fig. 1, Sphingomonas chlorophenolica or Sphingomonas herbicidovorans was considered to be most closely akin to the strain A0-1.

Thus, Sphingomonas chlorophenolica ATCC 33790 and Sphingomonas herbicidovorans DSM 11019 considered to be closely akin to A0-1 were hybridized, respectively. As shown in Table 1, the homology was 30% or less in any of the combination. It is therefore clear that the strain A0-1 is a novel species belonging to the genus Sphingomonas which is neither Sphingomonas chlorophenolica nor Sphingomonas herbicidovorans. Hereinafter the strain A0-1 is referred to as Sphingomonas sp. A0-1.

**Table 1**

| Strain | Sphingomonas sp. A0-1 | Sphingomonoas chlorophenolica ATCC33790 | Sphingomonas herbicidovorans DSM11019 |
|---|---|---|---|
| Sphingomonas sp.A0-1 | 100 | 5 | 4 |
| Sphingomonas chlorophenolica ATCC33790 | 17 | 100 | NT |
| Sphingomonas herbicidovorans DSM11019 | 30 | NT | 100 |

### EXAMPLE 2

### Degradation of bisphenol A by Sphingomonas sp. A0-1 and by bacteria akin thereto in BSM medium

Colonies of Sphingomonas sp. A0-1, Sphingomonas chlorophenolica ATCC 33790 and Sphingomonas herbicidovorans DSM 11019 were fished from MYPG agar medium (containing 3.0 g/L malt extract, 3.0 g/L yeast extract, 5.0 g/L polypeptide, 10 g/L glucose and 15 g/L agar, pH 7.0) and inoculated in 7 ml of MYPG medium charged in a test tube of 18 mm diameter. After shaking at 30°C for 17 hours at 100 rpm, the culture medium was centrifuged at 3000 rpm for 10 minutes to recover the bacterial mass. The recovered bacterial mass was washed twice with 50 mM phosphate buffer (pH 7.2) and suspended in physiological saline to give a bacterial cell suspension. The cell suspension was mixed with BSM medium (1.0 g/L K₂HPO₄, 1.0 g/L (NH₄)₂SO₄, 0.2 g/L MgSO₄·7H₂O, 0.01 g/L FeCl₃, 0.05 g/L NaCl and 0.05 g/L CaCl₂) supplemented with 100µg/mL of bisphenol A and 0.1% glucose, which was charged in a 300 ml Erlenmeyer flask, in such a ratio that the resulting mixture showed absorbance of 0.05 at 600 nm.

The flask was shaken at 30°C for 60 hours, while 1.5 ml each of a sample was collected every 12 hours. After the bacterial mass was removed by high speed centrifugation (15000 rpm, 5 mins.), bisphenol A in the medium was quantitatively determined using high performance liquid chromatography (Alliance 2690, made by Waters Inc.) under the following conditions; column: Mightysil RP18 GP150-4.6 (5µm), made by Kanto Kagaku K.K., eluate: 70% acetonitrile aqueous solution, column temperature: 40°C. Using a photo diode array as a detector, bisphenol A was quantitatively determined at absorbance of 217 nm.

As shown in Fig. 2, neither Sphingomonas chlorophenolica ATCC 33790 nor Sphingomonas herbicidovorans DSM 11019 showed any significant activity for the bisphenol A degradation but Sphingomonas sp. A0-1 was capable of completely degrading bisphenol A in about 48 hours.

### EXAMPLE 3

### Degradation of bisphenol A by Sphingomonas sp. A0-1 and by bacteria akin thereto in MYPG medium

In EXAMPLE 2, the cell suspension was suspended in 100 ml of BSM medium supplemented with bisphenol A and glucose. However, since the bacteria grew slowly in the BSM medium, in this EXAMPLE the same procedure as in EXAMPLE 2 was repeated using MYPG medium more abundant in nutrients. That is, a bacterial cell suspension was prepared from the culture medium obtained through preincubation in a manner similar to EXAMPLE 2. The cell suspension was mixed with 100 ml of MYPG medium supplemented with 100µg/mL bisphenol A in such a ratio that the resulting mixture showed absorbance of 0.05 at 600 nm. While shaking at 30°C for 36 hours, sampling was appropriately proceeded. Bisphenol A was quantitatively determined as in EXAMPLE 2.

As shown in Fig. 3, neither Sphingomonas chlorophenolica ATCC 33790 nor Sphingomonas herbicidovorans DSM 11019 exhibited any activity for bisphenol A degradation similarly in EXAMPLE 2 but Sphingomonas sp. A0-1 was capable of completely degrading bisphenol A in about 12 hours.

### EXAMPLE 4

### Effects of kind of sugars supplemented to BSM medium and their quantities on degradation of bisphenol A by Sphingomonas sp. A0-1

In this EXAMPLE, it was examined if the degradation of bisphenol A by Sphingomonas sp. A0-1 would be affected depending upon kind of sugars supplemented to BSM medium and their quantities supplemented. Preincubation, preparation of a cell suspension and conditions for quantitative determination of bisphenol A were the same as those in EXAMPLE 2. The cell suspension thus obtained was mixed with 100 ml of BSM medium supplemented with 100µg/mL of bisphenol A and sugars described below in such a proportion that the mixture showed abosrbance of 0.05 at 600 nm. The kind and concentration of sugars added are (1) 0.01% glucose, (2) 0.1% glucose, (3) 1.0% glucose, (4) 0.1% sucrose, (5) 1.0% sucrose, (6) 0.1% dextrin and (7) 0.1% maltose. While shaking at 30°C for 44 hours, 1.5 ml each of a sample was collected every definite time period. Thus, bisphenol A in the medium was quantitatively determined in a manner similar to EXAMPLE 2.

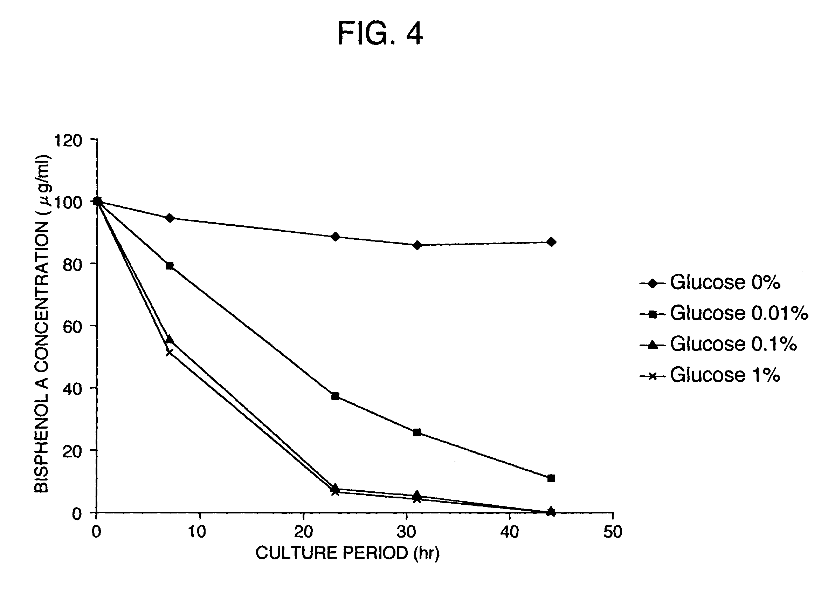

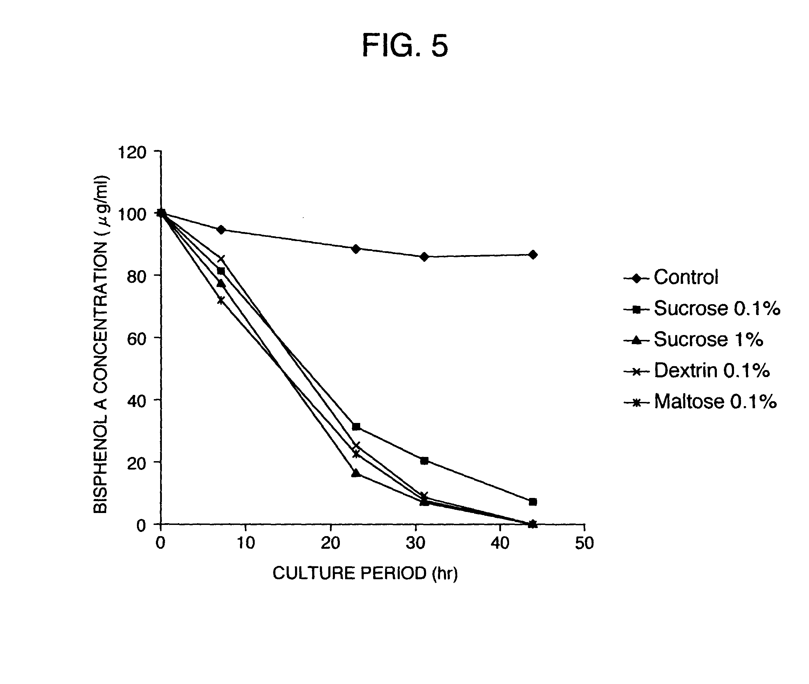

As shown in Figs. 4 and 5, the effect of accelerating the degradation of bisphenol A was noted by the addition of any of the sugars. Particularly when (2) 0.1% glucose or (3) 1.0% glucose was added to the medium, the degradation of bisphenol A proceeded most rapidly. Bisphenol A was decomposed to 6 to 8µg/mL in 23 hours after the culture and in 44 hours bisphenol A became completely undetectable.

### EXAMPLE 5

### Influence of culture temperatures on the growth of Sphingomonas sp. A0-1

A colony of Sphingomonas sp. A0-1 on MYPG agar medium was fished and inoculated on 7 ml of MYPG medium charged in a test tube in a diameter of 18 mm. After shake culture was performed at 30°C for 17 hours at 100 rpm, the culture medium was centrifuged at 3000 rpm for 10 minutes to recover the bacterial mass. The bacterial mass thus recovered was washed twice with 50 mM phosphate buffer (pH 7.2). The resulting cell suspension (2 - 3 x 10⁹ cfu/ml), 20µl, was added to 7 ml of MYPG medium charged in a test tube having a 18 mm diameter. Static culture was then conducted at every 5°C from 5°C to 45°C. Absorbance was measured at 600 nm after 3, 5 and 7 days.

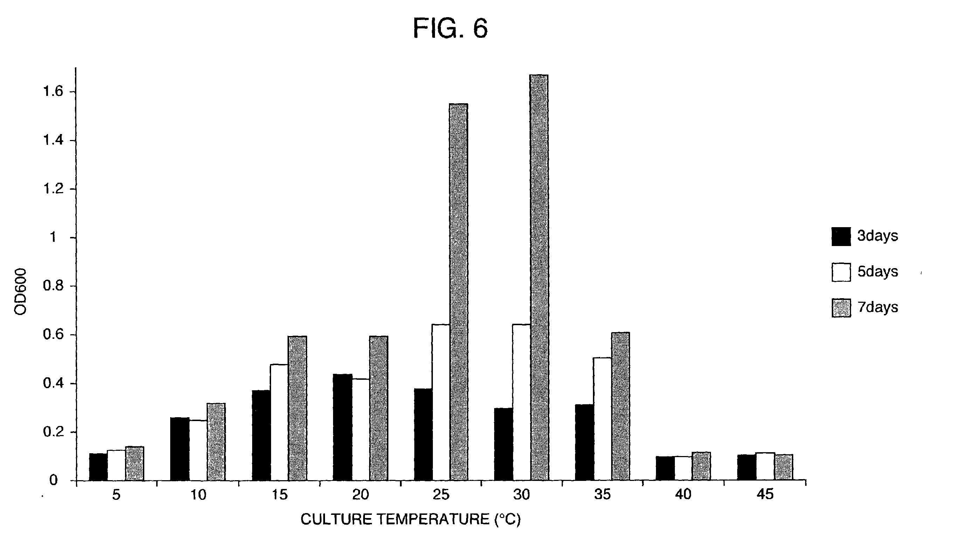

The results indicate that absorbance showed 0.076 with the medium alone but increased to 0.096 immediately after mixing with the bacterial mass. The relationship between culturing temperature, days for culturing and absorbance is shown in Fig. 6. The strain showed the fastest growth at 30°C but its growth was almost unappreciable at 40°C or higher. On the other hand, the strain hardly grew at 5°C.

### EXAMPLE 6

### Influence of medium pH values on the growth of Sphingomonas sp. A0-1

A colony of Sphingomonas sp. A0-1 on MYPG agar medium was fished and inoculated on 7 ml of MYPG medium charged in a test tube in a diameter of 18 mm. After shake culture was performed at 30°C for 17 hours at 100 rpm, the culture medium was centrifuged at 3000 rpm for 10 minutes to recover the bacterial mass. The bacterial mass thus recovered was washed twice with 50 mM phosphate buffer (pH 7.2). The resulting cell suspension (2 - 3 x 10⁹ cfu/ml), 20µl, was added to 7 ml each of MYPG medium charged in a test tube having a 18 mm diameter, the pH of each medium being adjusted with a difference by every pH 1 from pH 3 to 11. After shake culture was performed at 30°C for 7 hours, absorbance was measured at 600 nm. The absorbance shown in the results is the value obtained by subtracting the absorbance of the medium before the inoculation from that after the incubation.

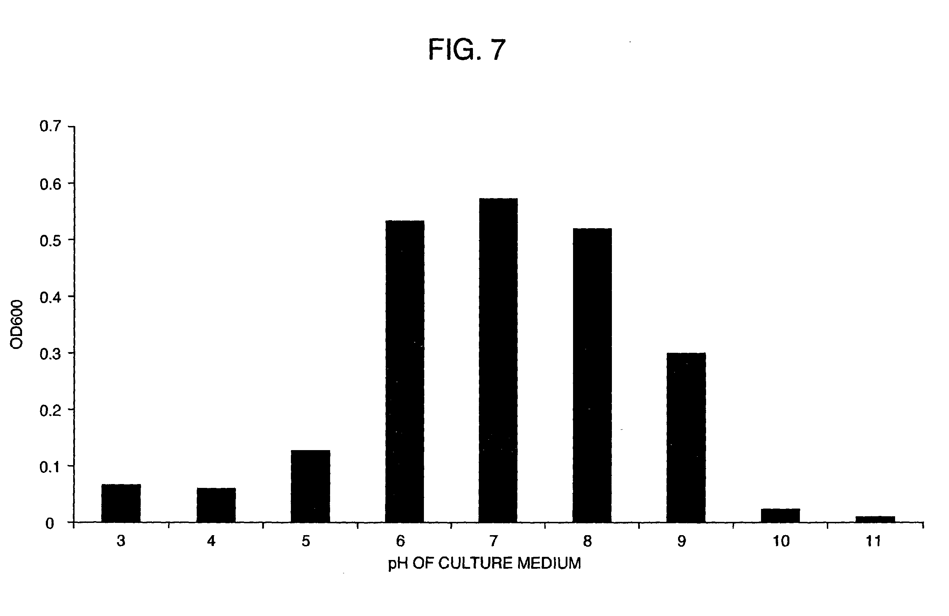

As shown in Fig. 7, the growth rate of the strain A0-1 reached the maximum at pH of 6 to 8, especially 7 of the medium. On the other hand, the strain did not grow appreciably at pH of 3, 4, 10 and 11.

### EXAMPLE 7

### Assimilation of Sphingomonas sp. A0-1 in various media

A colony of Sphingomonas sp. A0-1 on MYPG agar medium was fished and inoculated on 7 ml of a basal medium (0.05% yeast extract, 0.5% NaCl, 0.03% K₂HPO₄) supplemented with 0.2% each of sugars, alcohols, organic acids or amino acids shown in the table below for assimilation test. The substances tested are arabinose, fructose, glucose, lactose, maltose, sucrose, mannitol, sorbitol, inositol, methanol, erythritol, maleate, succinate, gluconate, tartarate, DL-alanine, L-histidine, L-valine, betaine, glycine, L-arginine and L-tryptophan. After shake culture was conducted at 30°C for 72 hours, Blue Thymol Blue (BTB) was added to the culture. When the color changed from blue to yellow or green, the microorganism was judged to secret an acid, indicating that assimilation was positive.

The results are shown in Table 2 below.

**Table 2:**

| Assimilation of Strain A0-1 | |
|---|---|
| Compound tested (0.2%) | Assimilation |
| Arabinose | + |
| Fructose | - |
| Glucose | + |
| Lactose | - |
| Maltose | + |
| Sucrose | + |
| Mannitol | - |
| Sorbitol | - |
| Inositol | - |
| Methanol | - |
| Erythritol | - |
| Maleate | - |
| Citrate | - |
| Gluconate | - |
| Lactate | - |
| DL-Alanine | - |
| L-Histidine | - |
| L-Valine | + |
| Betaine | - |
| Glycine | - |
| L-Arginine | - |
| L-Tryptophan | + |
| Control (basal medium alone) | - |

### EXAMPLE 8

### Physical adsorption of bisphenol A to the cell surface by Sphingomonas sp. A0-1 and uptake into the bacterial cell

The results of EXAMPLES 2 and 3 described above revealed that the level of bisphenol A in the medium was reduced. However, it is necessary to verify if bisphenol A was actually degraded or bisphenol A was merely adhered to the cell surface. Or, bisphenol A might be taken up into the bacterial cell during the growth of the strain A0-1. The following test was carried out to clarify the foregoing questions.

Preincubation and preparation of a cell suspension were carried out in a manner similar to EXAMPLE 2. The resulting cell suspension (cell density of 2 x 10¹⁰ cfu/ml) was mixed with 100 ml of BSM medium supplemented with 100µg/mL of bisphenol A and 0.1% glucose, in such a proportion that the resulting mixture showed absorbance of 0.05 at 600 nm. The cell suspension was treated at 100°C for 10 minutes to prepare a sterilized bacterial cell suspension. The same volume of the sterilized cell suspension as that of the cell suspension was mixed with 100 ml of the BSM medium described above.

With 100 ml of MYPG medium supplemented with 100 µg/mL of bisphenol A, which is more abundant in nutrients than BSM medium, the same test was performed using the cell suspension and the sterilized cell suspension.

The media after mixing were shake cultured at 30°C for 60 hours, during which 1.5 ml each of a sample was collected every 3-12 hours. In a manner similar to EXAMPLE 2, the cells were removed by centrifugation (15000 rpm, 10 mins.) and then bisphenol A in the medium was quantitatively determined. On the other hand, for quantitative determination of bisphenol A in the bacterial cells, the cell pellet obtained by the centrifugation was washed twice with 50 mM phosphate buffer (pH 7.2) and extracted with 1 ml of toluene. After the solvent was completely evaporated off, the residue was suspended in 1.5 ml of water to make a cell extract. The cell extract was provided for quantitative determination of bisphenol A.

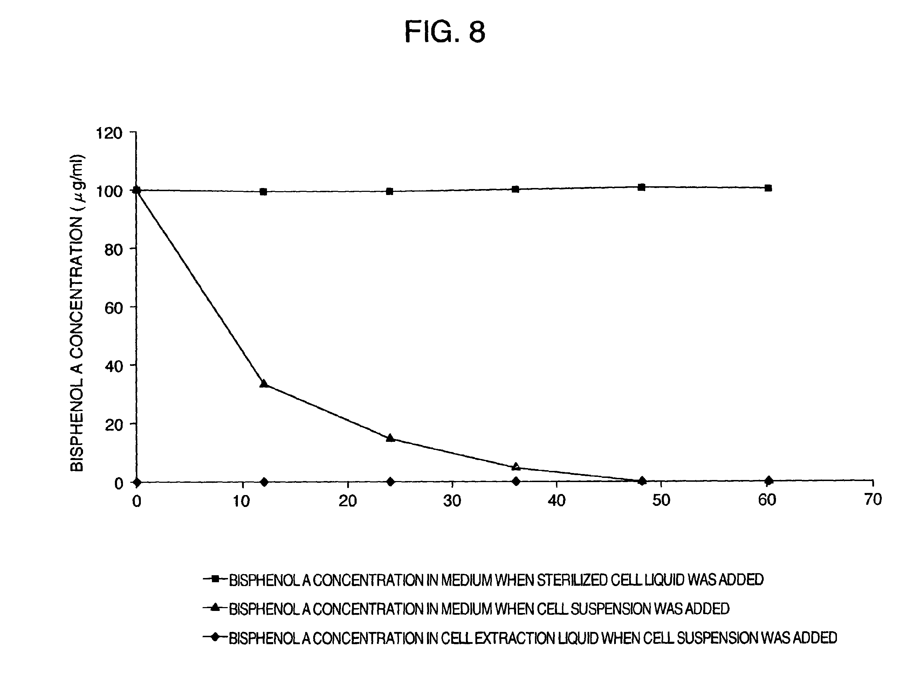

The results are shown in Figs. 8 (BSM medium) and 9 (MYPG medium). In both cases, the quantity of bisphenol A in the culture medium did not decrease at all even when the cell suspension sterilized by boiling was added to the culture medium. No bisphenol A was detected at all from the cell extract. The results suggest that in this test system, physical adsorption of the strain A0-1 onto the cell surface was extremely slight and bisphenol A was hardly taken up into the cell. Thus, the fact that the amount of bisphenol A decreased in the culture medium suggests that bisphenol A would be degraded or decomposed.

### EXAMPLE 9

### Resistance of Sphingomonas sp. A0-1 to organic solvent

Where the aquatic system containing bisphenol A is treated with a microorganism, the water to be treated generally contains other organic solvents. Therefore, resistance of this strain to organic solvents was examined. One platinum loop of colonies was fished from the plate medium of the strain and subjected to stroke culture on MYPG agar medium (with the components described above, 1.5% agar) solidified on a glass Petri dish in a 90 mm diameter. Stroke culture was carried out at 30°C for 24 hours to grow the strain. Thereafter, about 20 ml each of n-hexane, cyclohexane, p-xylene, o-xylene, toluene and n-heptanol was poured onto the Petri dish to fully overlay the grown cells. After the medium was then maintained at 30°C for 19 hours, the organic solvent in the Petri dish was discarded and the surface was air-dried. Using a platinum loop, the thus treated bacterial mass was transplanted onto a fresh MYPG agar medium followed by incubation at 30°C for 24 hours. The treated bacterial mass was judged to be alive or dead by the presence or absence of its growth.

As shown in Table 3 below, the results reveal that the strain A0-1 is resistant to n-hexane and cyclohexane.

**Table 3:**

| Resistance of strain A0-1 to organic solvent | | | | | | |
|---|---|---|---|---|---|---|
| Organic Solvent | n-Hexane | Cyclohexane | p-Xylene | o-Xylene | Toluene | n-Heptanol |
| Growth | + | + | - | - | - | - |

### EXAMPLE 10

### Induction of degradation enzyme by bisphenol A

In terms of more effective application, it is necessary to explore if a degradation enzyme is secreted (constitutive) whenever the strain A0-1 is used to decompose harmful substances such as bisphenol A or, if such an enzyme is secreted only by recognizing that a substance like bisphenol A is present (inductive). The following test was performed to clarify this regard.

A colony of Sphingomonas sp. A0-1 on MYPG agar medium was fished and inoculated on 7 ml of (1) MYPG medium and (2) MYPG medium supplemented with 100µ g/mL of bisphenol A, charged in a test tube with a 18 mm diameter, respectively. Preincubation was conducted by shaking at 30°C for 17 hours at 100 rpm. The culture medium was by centrifuged at 3000 rpm for 10 minutes to recover bacterial mass. The bacterial mass was washed twice with 50 mM phosphate buffer (pH 7.2) and suspended in physiological saline to produce a cell suspension. The thus obtained cell suspensions (1) and (2) were mixed with 100 ml of BSM medium supplemented with 100µg/mL of bisphenol A and 0.1% glucose, charged in a 300 ml Erlenmeyer flask, in such a ratio that the resulting mixture showed 0.05 of absorbance at 600 nm. While shaking at 30°C for 36 hours, 1.5 ml each of a sample was collected every 2 hours. Bisphenol A in the medium was quantitatively determined in a manner similar to EXAMPLE 2.

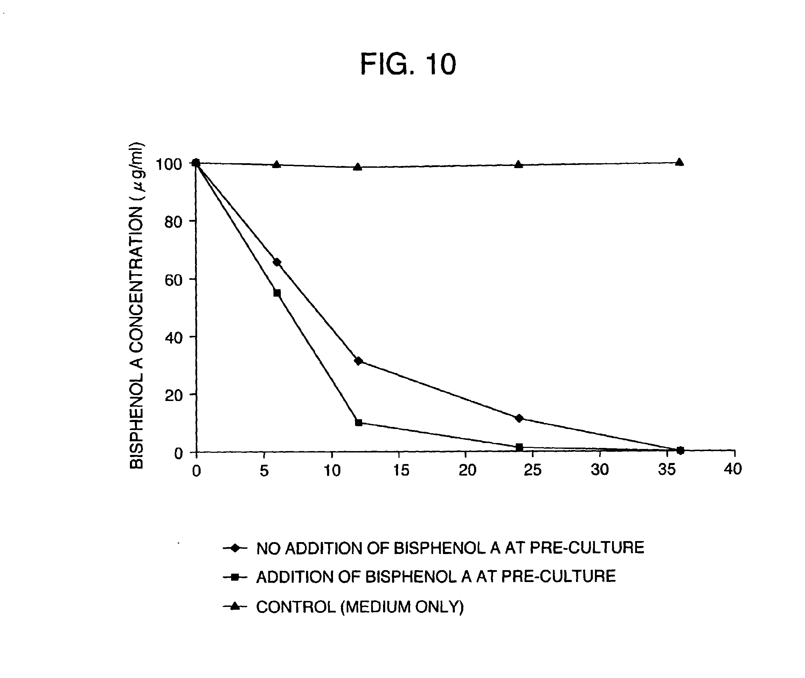

As shown in Fig. 10, the results clearly indicate that by adding bisphenol A to the culture medium during the preincubation stage, the degradation activity afterwards can be improved.

### EXAMPLE 11

### Degradation of bisphenol A by the culture filtrate of Sphingomonas sp. A0-1

Sphingomonas sp. A0-1 was cultured and then sterilized. The degradation of bisphenol A by the sterilized culture filtrate was examined.

A colony of Sphingomonas sp. A0-1 on MYPG agar medium was fished and inoculated on 7 ml of MYPG medium charged in a test tube with a 18 mm diameter. Shake culture was conducted at 30°C for 17 hours at 100 rpm. By subsequent centrifugation (15000 rpm, 10 mins.) followed by sterilization through filtration, the culture filtrate was obtained. Bisphenol A was added to 5 ml of the culture filtrate to reach a bisphenol A level of 100 µg/mL. While the temperature of the mixture was maintained from 25 to 50°C with a difference of every 5°C for 168 hours, about 1.5 ml each of a sample was collected from each system. Thereafter bisphenol A in the filtrate was quantitatively determined as in EXAMPLE 2.

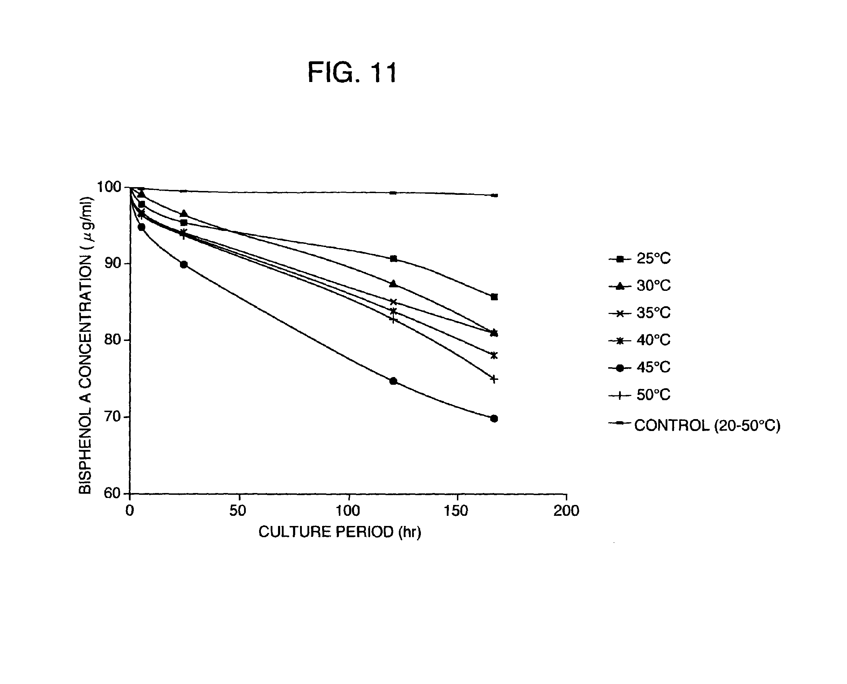

As shown in Fig. 11, the results reveal that the culture filtrate of Sphingomonas sp. A0-1 exhibits the activity of degrading bisphenol A and the highest activity can be obtained at 45°C but the degradation activity is slower than the case in which the living bacteria are growing.

### EXAMPLE 12

### Degradation of bisphenol A by the culture filtrate of Sphingomonas sp. A0-1 after lyophilization

Sphingomonas sp. A0-1 was cultured, sterilized and then lyophilized. The degradation activity of bisphenol A was examined with the sterilized lyophilized filtrate.

The culture filtrate was obtained in a manner similar to EXAMPLE 11. The thus obtained culture filtrate was stored in a refrigerator at 4°C for 48 hours and at -80°C for 48 hours. Bisphenol A was added to 5 ml of the stored filtrate to reach a bisphenol A level of 100µg/mL. While the mixture was maintained at 40°C for 120 hours, about 1.5 ml each of a sample was collected for sampling. Bisphenol A was quantitatively determined as in EXAMPLE 2.

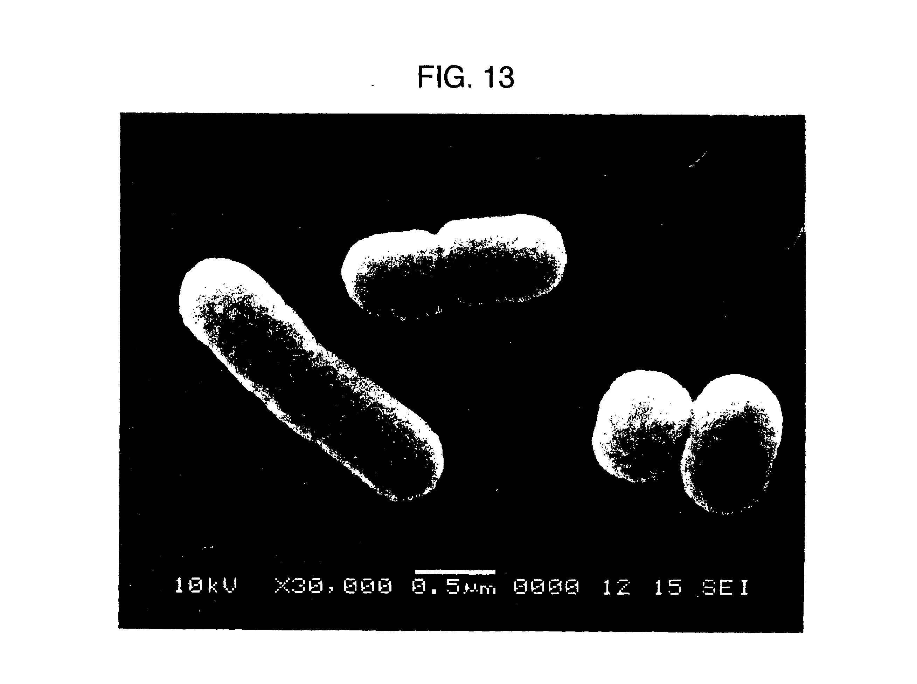

As shown in Fig. 13, the degradation of bisphenol A by the culture filtrate was almost equal to that of the culture filtrate prior to the storage, even after storage in a refrigerator at 4°C and at -80°C.

### EXAMPLE 13

### Scanning electromicroscopic photograph of Sphingomonas sp. A0-1

A scanning electromicroscopic photograph of Sphingomonas sp. A0-1 was taken by the following procedure. A colony of Sphingomonas sp. A0-1 on MYPG agar medium was fished and inoculated on 7 ml of MYPG medium charged in a test tube with a 18 mm diameter. Shake culture was conducted at 30°C for 17 hours at 100 rpm. The culture medium was subsequently centrifuged (15000 rpm, 10 mins.) to recover the bacterial mass. After fixing with 2% glutaraldehyde-supplemented 0.01M cacodylate buffer (NaCl, 0.15M) for an hour, the fixed mass was then washed with cacodylate buffer. Fixing was again performed with 2% osmate-supplemented 0.01M cacodylate buffer (NaCl, 0.15M) for an hour. After washing with distilled water, the concentration of ethanol was gradually elevated from 50% finally to 100%. The bacteria were lyophilized in a tert-butanol solution and then observed at an accelerated voltage of 10 KV in a high vacuum mode, using a scanning electromicroscopy JSM-5600LV, manufactured by Japan Electron Optics Laboratory Co., Ltd. The scanning electromicroscopic picture is shown in Fig. 14.

According to the present invention, there are provided novel microorganisms capable of degrading bisphenol A and the aromatic compounds having a stilbene skeleton more efficiently than hitherto known bacteria. By using the microorganisms of the invention, bisphenol A and the aromatic compounds having a stilbene skeleton can be degraded or decomposed very efficiently. Therefore, the invention can be extensively applied to the environmental cleanup.

## Claims

1. A novel bacterium capable of degrading at least one aromatic compound selected from bisphenol A and an aromatic compound containing a stilbene skeleton, belonging to the genus Sphingomonas but classified into no known species.

2. A bacterium according to claim 1, which has the following properties.
| | |
|---|---|
| Morphology | rod |
| Gram staining | - |
| Spore formation | - |
| Motility | + |
| Behavior to oxygen | aerobic |
| Oxidase test | + |
| Catalase test | + |
| OF | - |
| Color of colonies | yellowish |

3. A bacterium according to claim 1 or 2, which is Sphingomonas sp. A0-1 (FERM BP-7435).

4. A method for degradation of an aromatic compound which comprises allowing a bacterium according to any one of claims 1 to 3 to act on an aromatic compound selected from bisphenol A and an aromatic compound containing a stilbene skeleton.

5. A method for degradation of an aromatic compound in soils, waters or other wastes containing at least one aromatic compound selected from bisphenol A and an aromatic compound containing a stilbene skeleton which comprises adding a culture of a bacterium according to any one of claims 1 to 3 or a carrier bearing a mass of the bacterium to the soils, waters or other wastes.

6. A method for degradation according to claim 5, wherein said culture is a living bacterial mass.

7. A method for degradation according to any one of claims 5 and 6, wherein said culture is a sterilized culture.
